Europäisches Patentamt

(19) European Patent Office

Office européen des brevets

(11) Publication number : **0 291 117 B1**

(12) **EUROPEAN PATENT SPECIFICATION**

(45) Date of publication of patent specification :
15.01.92 Bulletin 92/03

(51) Int. Cl.⁵ : **C07C 69/24,** C07C 67/38,
C07C 53/122, C07C 51/14,
C01G 55/00

(21) Application number : 88200871.7

(22) Date of filing : 03.05.88

(54) **Process for the preparation of carboxylic acids or of esters thereof.**

The file contains technical information
submitted after the application was filed and
not included in this specification

(30) Priority : 15.05.87 GB 8711524

(43) Date of publication of application :
17.11.88 Bulletin 88/46

(45) Publication of the grant of the patent :
15.01.92 Bulletin 92/03

(84) Designated Contracting States :
BE DE ES FR GB IT NL

(56) References cited :
EP-A- 0 105 704
DE-A- 2 060 863
DE-A- 2 948 888

(56) References cited :
US-A- 4 256 913
GMELINS HANDBUCH DER ORGANISCHEN
CHEMIE, 8th edition, system no. 63:
"Ruthenium", 1938; VERLAG CHEMIE GMBH,
Berlin, page 8

(73) Proprietor : SHELL INTERNATIONALE
RESEARCH MAATSCHAPPIJ B.V.
Carel van Bylandtlaan 30
NL-2596 HR Den Haag (NL)

(72) Inventor : Drent, Eit
Badhuisweg 3
NL-1031 CM Amsterdam (NL)

(74) Representative : Tuijn, Jan Warnaar
Shell Internationale Research Maatschappij
B.V., Patents, Licensing & Trade Marks
Division, P.O. Box 302
NL-2501 CH The Hague (NL)

EP 0 291 117 B1

## Description

The invention relates to a process for the preparation of carboxylic acids or of esters thereof. The invention also relates to a novel composition.

U.S. patent specification 3,168,553 discloses a process in which olefins are carbonylated in the presence of a complex comprising a trialkylphosphine together with cobalt, ruthenium, rhodium or iridium. However, this process requires the use of high pressures, and its selectivity towards the desired product is often unsatisfactory. For instance, the carbonylation of ethylene in the presence of ethanol and $CO_2(CO)_8$ as the catalyst leads to the formation not only of ethyl propionate, but also of large quantities of by-products, such as diethyl ketone and acetaldehyde.

It has now surprisingly been found that carboxylic acids or esters thereof are formed at a very high selectivity at relatively low pressure by contacting an olefinically unsaturated compound with carbon monoxide in the presence of water or an alcohol, respectively, and in the presence of a ruthenium compound and a compound defined more closely hereinafter.

The invention therefore provides a process for the preparation of carboxylic acids or of esters thereof which process comprises contacting an olefinically unsaturated compound with carbon monoxide in the presence of water or an alcohol, respectively, and of a catalytic system which may be prepared by combining the following components :

component (a) — a ruthenium compound, and
component (b) — a compound having a non-coordinating anion of an acid with a $pK_a$, measured at 25°C in aqueous solution, below 0.5.

The selectivity to a certain compound, expressed in a percentage, is defined herein as $100 \times c : d$ in which "c" is the amount of starting olefinically unsaturated compound that has been converted into that certain compound and "d" is the total amount of starting olefinically unsaturated compound that has been converted.

It is a feature of the present invention that the catalytic system is very stable and, therefore, can be used for a long time :

no plating out of metallic ruthenium has been observed. Replacement of component (a) with a palladium compound would result in plating out of metallic palladium. EP-A-105704 discloses a carbonylation process using palladium catalysts, wherein either oxygen addition or incremental Cu(II) addition is practised preventing palladium reduction. However, this process requires high palladium concentrations for achieving unsatisfactory reaction rates.

The acids have a non-coordinating anion, by which is meant that little or no co-valent interaction takes place between the ruthenium and the anion. Component (b) preferably comprises a compound having an anion of sulphuric acid, of a sulphonic acid or of an acid that can be formed by interaction of a Lewis acid with a Broensted acid, which formation may take place in-situ. Examples of such Lewis acids are $BF_3$, $AsF_5$, $SbF_5$, $PF_5$, $TaF_5$ and $NbF_5$ and examples of such Broensted acids are hydrohalogenic acids, in particular HF and HCl. Component (b) in particular comprises a compound having an anion of trifluoromethanesulphonic acid $[CF_3SO_2(OH)]$, of tetrafluoroboric acid ($HBF_4$) or of hexafluorophosphoric acid ($HPF_6$). Other examples of component (b) are compounds having an anion of perchloric acid ($HClO_4$), fluorosilicic acid ($H_2SiF_6$), fluorosulphonic acid $[SO_2(OH)F]$ and chlorosulphonic acid $[SO_2(OH)Cl]$.

The compound having a non-coordinating anion of an acid with a $pK_a$ below 0.5 may be a salt or an acid; component (b) may be a mixture of such a salt and such an acid. The salts used as component (b) may have a great variety of cations. Very good results have been obtained with cations of transition metals, particularly zinc and copper. Very good results have also been obtained with uranyl salts. Components (a) and (b) may be the same, i.e. when the ruthenium compound has a non-coordinating anion, as is the case with $Ru(PF_6)_3$.

Examples of suitable ruthenium compounds are ruthenium oxides and ruthenium salts of acids having a non-coordinating anion as specified hereinbefore. Very good results have been obtained with ruthenium tris acetylacetonate.

The quantity of component (a) used may vary within wide ranges and is generally in the range between $10^{-6}$ and $10^{-1}$ and preferably between $10^{-5}$ and $10^{-2}$ gram-atom ruthenium per mol starting olefinically unsaturated compound.

The quantity of component (b) may also vary within wide ranges ; component (b) is preferably present in a quantity in the range of from 0.1 to 100 and particularly from 0.5 to 20 equivalents per gram-atom of ruthenium.

The process according to the present invention may be carried out at a temperature and a pressure which are not critical and may vary within wide ranges. The process is preferably carried out at a temperature in the range of from 100°C to 250°C and a pressure in the range of from 5 to 200 bar, but temperatures above or below said range or pressures above or below said range may also be used.

The olefinically unsaturated compound may be an unsubstituted or a substituted alkene or cycloalkene preferably having 2-30, and in particular 2-20, carbon atoms and preferably 1-3 double bonds. The alkene or cycloalkene may be substituted, for instance, with one or more halogen atoms or cyano, ester, alkoxy, hydroxy, carboxy or aryl groups. If the substituents are not inert under the reaction conditions, the carboxylation reaction may be accompanied with other reactions. For instance, the carbonylation of allyl alcohol is accompanied with esterification of the hydroxy group. Examples of suitable olefinic compounds are ethene, propene, 1-butene, 2-butene, isobutene, the isomeric pentenes, hexenes, octenes and dodecenes, cyclooctadiene-(1,5), cyclododecene, cyclododecatriene-(1,5,9), allyl alcohol, methyl acrylate, ethyl acrylate, methyl methacrylate, acrylonitrile, acrylamide, N,N-dimethylacrylamide, vinyl chloride, allyl chloride, acrolein, oleic acid, methyl allyl ether and styrene.

The alcohols used in the process according to the invention may be aliphatic, cycloaliphatic or aromatic and may be substituted with one or more substituents, such as mentioned hereinbefore in connection with the olefinically unsaturated compounds to be used as starting material. The alcohol may therefore also be a phenol.

The alcohols preferably contain not more than 20 carbon atoms. Examples of suitable alcohols are methanol, ethanol, propanol, isobutanol, tert.butyl alcohol, stearyl alcohol, benzyl alcohol, cyclohexanol, allyl alcohol, chlorocapryl alcohol, ethylene glycol, 1,2-propanediol, 1,4-butanediol, glycerol, polyethylene glycol, 1,6-hexanediol, phenol and cresol.

Special preference is given to alkanols having in the range of from 1 to 10 carbon atoms per molecule. If the alcohol has more than one hydroxy group, different products may be formed, depending on the molar ratios existing between the reagents. For instance, depending on the quantity of olefinically unsaturated compound used, either a mono-ester or a di-ester may be produced from glycerol. The process may be carried out in the simultaneous presence of water and an alcohol, in which case a carboxylic acid and an ester are simultaneously formed. The process according to the present invention is particularly suitable for the preparation of methyl propionate, starting from ethylene, carbon monoxide and methanol, methyl propionate being an important solvent.

In the process according to the invention the carbon monoxide may be used pure or diluted with an inert gas, such as nitrogen, noble gasses or carbon dioxide. Generally the presence of more than 10%v of hydrogen is undesirable. Generally preference is given to the use of carbon monoxide or a carbon monoxide-containing gas which contains less than 5%v of hydrogen.

The molar ratio of the olefinically unsaturated compound to water or alcohol is not critical. The molar ratio between hydroxy groups and olefinic double bonds may lie for instance between 0.1 : 1 and 10 : 1. When using a mono-olefin and either water or a monohydric alcohol preference is usually given to the use of an excess of the hydroxy compound mentioned. However, when using a polyhydric alcohol to prepare a polyester, it will generally be necessary to use an excess of olefinic compound.

The process according to the invention may be carried out batchwise, continuously or semi-continuously. Generally there is no need for the use of a solvent since usually there will be an excess of one of the reactants — for instance the alcohol — which may serve as a solvent as well. If required, however, a solvent may be used, for instance dimethyl sulphoxide, diisopropyl sulphone, tetrahydrothiophene 1,1-dioxide (also referred to as sulfolane), acetone, chloroform, methyl isobutyl ketone, diglyme (dimethyl ether of di-ethylene glycol) or diisopropyl ether. The primary reaction product of the carbonylation reaction may also be used as a solvent.

The reaction mixtures obtained may be subjected to suitable catalyst and product separating means comprising one or more such steps, for example, as stratification, solvent extraction, distillation, fractionation or adsorption. The catalytic system as well as unconverted starting compounds or solvent, if any, may be recycled, in part or entirely, to the reaction zone.

The novel composition referred to hereinbefore comprises the following components :

component (a) — a ruthenium compound, and
component (b) — a transition metal salt having a non-coordinating anion of an acid with a $pK_a$, measured at 25°C in aqueous solution, below 0.5.

The following Examples further illustrate the invention. The experiments were carried out in a 300 ml magnetically stirred Hastelloy C autoclave ("Hastelloy" is a trade mark). The reaction mixtures obtained were analyzed by means of gas-liquid chromatography.

## Examples 1-14

The autoclave was charged with methanol (50 ml, 1.23 mol), ruthenium(acetylacetonate)$_3$ (0.5 mmol) and a compound having an anion of an acid with a $pK_a$ below 0.5, flushed with carbon monoxide, sealed, charged with carbon monoxide until a partial pressure thereof of 25 bar was obtained, charged with ethylene until a partial pressure thereof of 25 bar was obtained and heated. After a reaction time of 5 h the autoclave was allowed to adopt ambient temperature and opened for analysis of the reaction mixture.

Table 1 hereinafter shows for each of the Examples which compound having an anion of an acid having a $pK_a$ below 0.5 was used — referred to as "component (b)" —, in which amount this compound was used and the temperature to which the autoclave was heated. Table 1 also presents the reaction rates and the selectivities to methyl propionate.

EP 0 291 117 B1

## TABLE 1

| Example | Component (b) | mmol | Temperature °C | Reaction rate, mol $C_2H_4$ per gram-atom Ru per h | Selectivity, % to methyl propionate |
|---------|---------------|------|----------------|-----------------------------------------------------|--------------------------------------|
| 1 | $UO_2SO_4$ | 1 | 150 | 20 | 92 |
| 2 | ditto | 1 | 160 | 35 | 93 |
| 3 | ditto | 1 | 180 | 100 | 92 |
| 4 | $ZnSO_4$ | 1 | 160 | 20 | 94 |
| 5 | $CuSO_4$ | 1 | 160 | 20 | 92 |
| 6 | $H_2SO_4$ | 2 | 150 | 15 | 76 |
| 7 | $Zr(SO_4)_2$ | 1 | 150 | 10 | 75 |
| 8 | $Cu(BF_4)_2$ | 1 | 160 | 20 | 93 |
| 9 | ditto | 1 | 175 | 50 | 92 |
| 10 | $HBF_4$ | 1 | 175 | 40 | 97 |
| 11 | $HPF_6$ | 1 | 175 | 80 | 93 |
| 12 | ditto | 2 | 175 | 100 | 93 |
| 13 | $Cu(PF_6)_2$ | 2 | 175 | 120 | 94 |
| 14 | $CF_3SO_3H$ | 2 | 150 | 10 | 85 |

Comparative Experiments A-C

These three experiments were carried out as described in Examples 1-14 with the exception of the replacement of component (b) mentioned in Table 1 with the compound having an anion of an acid with a $pK_a$ above 0.5 ; this compound and the temperature of the experiment are stated in Table 2. The selectivities to methyl propionate found in these experiments were considerably lower than these presented in Table 1 which resulted from examples where compounds having an anion of an acid with a $pK_a$ below 0.5 were applied.

TABLE 2

| Comparative Experiment | Additional Component | mmol | Temperature °C | Reaction rate, mol $C_2H_4$ per gram - atom Ru per h | Selectivity, % to methyl propionate |
|---|---|---|---|---|---|
| A | p-toluenesulphonic acid | 2 | 150 | 10 | 65 |
| B | cupric(tosylate)$_2$ | 1 | 150 | 15 | 50 |
| C | ditto | 1 | 175 | 50 | 60 |

EP 0 291 117 B1

Comparative Experiment D

Example 1 was repeated with the difference that no $UO_2SO_4$ was present. No reaction was observed.

Example 15

Example 8 was repeated with the difference that also 0.5 mmol of 1,3-di(diphenylphosphino)propane was present. The reaction rate was 15 mol ethylene per gram-atom ruthenium per h and the selectivity to methyl propionate was 81%. Comparison with the results of Example 8 shows that reaction rate and selectivity are higher in the absence of 1,3-di(diphenylphosphino)propane.

Example 16

The autoclave was charged with methanol (50 ml), ruthenium(acetylacetonate)$_3$ (0.5 mmol) and cupric hexafluorophosphate (2 mmol), flushed with carbon monoxide, sealed, charged with carbon monoxide until a partial pressure thereof of 40 bar was obtained, charged with propylene (30 ml) and heated to a temperature of 175°C. After a reaction time of 5 h the autoclave was allowed to adopt ambient temperature and opened for analysis of the reaction mixture.

The reaction rate was 40 mol propylene per gram-atom ruthenium per h and the selectivity to the total methyl butyrate and methyl isobutyrate was 96% in a ratio of 58 : 42, respectively.

Example 17

The autoclave was charged with diglyme (40 mol), ruthenium(acetylacetonate)$_3$ (0.5 mmol), hexafluorophosphoric acid (1.5 mmol) and water (10 ml), flushed with carbon monoxide, sealed, charged with carbon monoxide until a partial pressure thereof of 25 bar was obtained, charged with ethylene until a partial pressure thereof of 25 bar was obtained and heated to a temperature of 160°C. After a reaction time of 5 h the autoclave was allowed to adopt ambient temperature and opened for analysis of the reaction mixture.

The reaction rate was 65 mol ethylene per gram-atom ruthenium per h and the selectivity to propionic acid was 95%.

Example 18

The autoclave was charged with water (50 ml), ruthenium(acetylacetonate)$_3$ (0.5 mmol) and cupric hexafluorophosphate (2 mmol), flushed with carbon monoxide, sealed, charged with carbon monoxide until a partial pressure thereof of 25 bar was obtained, charged with ethylene until a partial pressure thereof of 25 bar was obtained and heated to a temperature of 160°C. After a reaction time of 5 h the autoclave was allowed to adopt ambient temperature and opened for analysis of the reaction mixture.

The reaction rate was 40 mol ethylene per gram-atom ruthenium per h and the selectivity to propionic acid was 90%.

This example shows that water may be used as a solvent.

**Claims**

1. A process for the preparation of carboxylic acids or of esters thereof which process comprises contacting an olefinically unsaturated compound with carbon monoxide in the presence of water or an alcohol, respectively, and of a catalytic system which may be prepared by combining the following components :

component (a) — a ruthenium compound, and
component (b) — a compound having a non-coordinating anion of an acid with a $pK_a$, measured at 25°C in aqueous solution, below 0.5.

2. A process as claimed in claim 1 in which component (a) comprises ruthenium tris acetylacetonate.

3. A process as claimed in claim 1 or 2 in which component (b) comprises a compound having an anion of sulphuric acid, of a sulphonic acid or of an acid that can be formed by interaction of a Lewis acid with a Broensted acid.

4. A process as claimed in claim 3 in which component (b) comprises a compound having an anion of trifluoromethanesulphonic acid, of tetrafluoroboric acid or of hexafluorophosphoric acid.

5. A process as claimed in any one of the preceding claims in which component (b) is a salt of a transition metal.

6. A process as claimed in claim 5 in which the transition metal is zinc or copper.

7. A process as claimed in any one of the preceding claims in which component (b) is present in a quantity in the range of from 0.1 to 100 equivalents per gram-atom of ruthenium.

8. A process as claimed in any one of the preceding claims which is carried out at a temperature in the range of from 100°C to 250°C and a pressure in the range of from 5 to 200 bar.

9. A process as claimed in any one of the preceding claims in which the olefinically unsaturated compound is an alkene having in the range of from 2 to 30 carbon atoms per molecule.

10. A composition comprising the following components :

| | |
|---|---|
| component (a) — | a ruthenium compound, and |
| component (b) — | a transition metal salt having a non-coordinating anion of an acid with a $pK_a$, measured at 25°C in aqueous solution, below 0.5. |

**Patentansprüche**

1. Verfahren zur Herstellung von Carbonsäuren oder Estern davon, wobei das Verfahren umfaßt das Zusammenbringen einer olefinisch ungesättigten Verbindung mit Kohlenmonoxid in Gegenwart von Wasser bzw. einem Alkohol, und einem katalytischen System, das hergestellt werden kann durch Zusammenbringen der folgenden Komponenten :

| | |
|---|---|
| Komponente (a) — | eine Rutheniumverbindung und |
| Komponente (b) — | eine Verbindung mit einem nicht-koordinierenden Anion einer Säure mit einem $pK_a$-Wert, gemessen bei 25°C in wässriger Lösung, unter 0,5. |

2. Verfahren nach Anspruch 1, bei dem die Komponente (a) Rutheniumtrisacetylacetonat umfaßt.

3. Verfahren nach Anspruch 1 oder 2, wobei die Komponente (b) eine Verbindung mit einem Anion von Schwefelsäure, von einer Sulfonsäure oder von einer Säure die gebildet werden kann durch Wechselwirkung einer Lewis-Säure mit einer Broensted-Säure, umfaßt.

4. Verfahren nach Anspruch 3, wobei die Komponente (b) eine Verbindung mit einem Anion von Trifluormethansulfonsäure, von Tetrafluorborsäure oder von Hexafluorphosphorsäure umfaßt.

5. Verfahren nach einem der vorangehenden Ansprüche, wobei die Komponente (b) ein Salz eines Übergangsmetalls ist.

6. Verfahren nach Anspruch 5, wobei das Übergangsmetall Zink oder Kupfer ist.

7. Verfahren nach einem der vorangehenden Ansprüche, wobei die Komponente (b) in einer Menge im Bereich von 0,1 bis 100 Äquivalent pro g-Atom Ruthenium vorhanden ist.

8. Verfahren nach einem der vorangehenden Ansprüche, das bei einer Temperatur im Bereich von 100°C bis 250°C und einem Druck im Bereich von 5 bis 200 bar durchgeführt wird.

9. Verfahren nach einem der vorangehenden Ansprüche, wobei die olefinische ungesättigte Verbindung ein Alken mit 2 bis 30 Kohlenstoffatomen im Molekül ist.

10. Masse, umfassend die folgenden Komponenten :

| | |
|---|---|
| Komponente (a) — | eine Rutheniumverbindung und |
| Komponente (b) — | ein Übergangsmetall-Salz mit einem nicht-koordinierendem Anion einer Säure mit einem $pK_a$-Wert, gemessen bei 25°C in wässriger Lösung, unter 0,5. |

**Revendications**

1. Procédé de préparation d'acides carboxyliques ou de leurs esters, lequel procédé comprend la mise en contact d'un composé oléfiniquement insaturé avec du monoxyde de carbone, en présence d'eau et d'un alcool, respectivement, et d'un système catalytique que l'on peut préparer en combinant les composants suivants :

| | |
|---|---|
| Composant (a) — | un composé de ruthénium, et |
| Composant (b) — | un composé ayant un anion non coordinant d'un acide avec $pK_a$, mesuré à 25°C en solu- |

EP 0 291 117 B1

tion aqueuse, inférieur à 0,5.

2. Procédé selon la revendication 1, dans lequel le composant (a) comprend le tris-acétylacétonate de ruthénium.

3. Procédé selon la revendication 1 ou 2, dans lequel le composant (b) comprend un composé ayant un anion d'acide sulfurique, d'un acide sulfonique ou d'un acide qui peut être formé par interaction d'un acide de Lewis avec un acide de Broensted.

4. Procédé selon la revendication 3, dans lequel le composant (b) comprend un composé ayant un anion d'acide trifluorométhanesulfonique, d'acide tétrafluoroborique, ou d'acide hexafluorophosphorique.

5. Procédé selon l'une quelconque des revendications précédentes, dans lequel le composant (b) est un sel d'un métal de transition.

6. Procédé selon la revendication 5, dans lequel le métal de transition est le zinc ou le cuivre.

7. Procédé selon l'une quelconque des revendications précédentes, dans lequel le composant (b) est présent en une quantité située dans un intervalle allant de 0,1 à 100 équivalents par atome-gramme de ruthénium.

8. Procédé selon l'une quelconque des revendications précédentes, qui est conduit à une température située dans un intervalle allant de 100°C à 250°C et à une pression située dans un intervalle allant de 5 à 200 bar.

9. Procédé selon l'une quelconque des revendications précédentes, dans lequel le composé oléfiniquement insaturé est un alcène ayant de 2 à 30 atomes de carbone par molécule.

10. Composition comprenant les composants suivants :

Composant (a) — un composé de ruthénium, et

Composant (b) — un sel de métal de transition ayant un anion non coordinant d'un acide avec un $pK_a$, mesuré à 25°C, en solution aqueuse, inférieur à 0,5.